Europäisches Patentamt

European Patent Office

Office européen des brevets

(11) Veröffentlichungsnummer: **0 245 841**
**A2**

(12) **EUROPÄISCHE PATENTANMELDUNG**

(21) Anmeldenummer: **87106876.3**

(22) Anmeldetag: **12.05.87**

(51) Int. Cl.⁴: **C12G 3/08** , //C12N1/18

(30) Priorität: **13.05.86 DE 3616094**

(43) Veröffentlichungstag der Anmeldung:
**19.11.87 Patentblatt  87/47**

(84) Benannte Vertragsstaaten:
**AT BE CH DE ES FR GB GR IT LI LU NL SE**

(71) Anmelder: **Holsten-Brauerei AG**
**Postfach 50 07 49 Holstenstrasse 224**
**D-2000 Hamburg 50(DE)**

(72) Erfinder: **Dziondziak, Klaus, Dr.**
**Datumer Chaussee 106**
**D-2080 Pinneberg(DE)**

(74) Vertreter: **Vossius & Partner**
**Siebertstrasse 4 P.O. Box 86 07 67**
**D-8000 München 86(DE)**

(54) Verfahren zur Herstellung von alkoholarmen oder alkoholfreien Bieren.

(57) Gegenstand der Erfindung ist ein Verfahren zur Herstellung eines alkoholarmen oder alkoholfreien Bieres mit einem die Vollmundigkeit verbessernden Gehalt von 0,3 bis 2,0 Vol.% Glycerin. Im Verfahren der Erfindung wird die Gärung mit einer ADH-negativen, nicht zur Alkoholbildung, dafür aber zu vermehrter Glycerinbildung befähigten Hefemutante durchgeführt. Das erhaltene alkoholfreie Bier kann mit gewöhnlichem alkoholhaltigem Bier zu einem alkoholarmen Bier verschnitten werden.

EP 0 245 841 A2

## " Verfahren zur Herstellung von alkoholarmen oder alkoholfreien Bieren "

Die Erfindung betrifft ein Verfahren zur Herstellung von alkoholarmen oder alkoholfreien Bieren.

Es sind bereits zahlreiche Verfahren zur Herstellung von alkoholarmen und alkoholfreien Bieren bekannt. Zu diesem Zweck kann einem nach herkömmlichen Brauverfahren hergestellten Bier mit normalem Alkoholgehalt nachträglich wenigstens ein Teil des Alkohols entzogen werden. Das in der DE-A-14 42 238 beschriebene Verfahren dieser Art sieht ein Abdampfen des Alkohols in einem Dünnschichtverdampfer bei Temperaturen unterhalb von 70°C vor. Aus der DE-A-12 66 266 ist ein ähnliches Verfahren bekannt, bei welchem das Bier zunächst einer Zerstäubungsverdampfung im Vakuum und dann anschließend einer Dünnschichtverdampfung im Vakuum unterworfen und der Rückstand rückverschnitten und mit Kohlensäure imprägniert wird.

Aus der DE-A-24 05 543 und der DE-A-721 249 sind ferner Verfahren zur adsorbtiven Alkoholentfernung aus Bier bekannt. Ein Verfahren zur Herstellung von alkoholarmem Bier durch Umkehrosmose ist in der DE-A-23 23 094 offenbart. Schließlich wurden auch Verfahren vorgeschlagen, bei denen der Alkohol in der Sudpfanne ausgekocht wird.

Andererseits wurden Verfahren zur Herstellung von alkoholarmen und alkoholfreien Bieren entwickelt, die von vornherein eine verminderte Alkoholbildung während des Herstellungsprozesses vorsehen.

Dies wird entweder dadurch erreicht, daß man mit Hefen arbeitet, die die Bierwürze nur unvollkommen vergären können, oder man reprimiert bzw. unterbricht die Gärung; vgl. DE-A-520 363 und DE-A-728 871.

Insgesamt haftet den genannten Verfahren der Nachteil an, daß die daraus gewonnenen alkoholarmen oder alkoholfreien Biere geschmacklich nicht normalen alkoholhaltigen Bieren entsprechen. Die nachträglich entalkoholisierten Biere schmecken leer und unharmonisch, während die Biere, bei denen von vornherein die Alkoholbildung ausgeschlossen bzw. vermindert wurde, einen typischen unangenehmen Bierwürzgeschmack aufweisen. Hinzu kommt noch, daß die verwendeten Entalkoholisierungsverfahren sehr aufwendig und störanfällig sind.

In der am 11.6.1986 veröffentlichten europäischen Patentanmeldung 183 858 ist ein Bier, insbesondere ein alkoholarmes oder alkoholfreies Bier beschrieben, das gekennzeichnet ist durch einen die Vollmundigkeit verbessernden Gehalt von 0,3 bis 2,0 Vol.% Glycerin. Es wird ein Verfahren beschrieben, bei dem mittels vermehrt Glycerin-und Zuckeralkohole bildender Hefen eine Gärung durchgeführt wird, bei der gleichzeitig weniger Alkohol gebildet wird.

Gärungsverfahren, bei denen vermehrt Glycerin und praktisch kein Alkohol gebildet wird, sind nicht bekannt.

Der Erfindung liegt die Aufgabe zugrunde, ein abgewandeltes Verfahren zur Herstellung alkoholarmer oder alkoholfreier Biere zu schaffen, die einen die Vollmundigkeit verbessernden Gehalt von 0,3 bis 2,0 Vol.% Glycerin aufweisen. Diese Aufgabe wird durch den Einsatz einer zur Bierherstellung geeigneten Hefe bei der Gärung gelöst, die durch genetische Veränderungen nicht zur Alkoholbildung, dafür aber zur vermehrten Glycerinbildung befähigt ist. Für diesen Zweck geeignet sind ADH-negative, insbesondere ADH 1-negative Hefemutanten.

Gegenstand der Erfindung ist somit ein Verfahren zur Herstellung eines alkoholarmen oder alkoholfreien Bieres mit einem die Vollmundigkeit verbessernden Gehalt von 0,3 bis 2,0 Vol.% Glycerin, das dadurch gekennzeichnet ist, daß man die Gärung mit einer ADH-negativen, nicht zur Alkoholbildung, dafür aber zu vermehrter Glycerinbildung befähigten Hefemutante durchführt, und das erhaltene alkoholfreie Bier gegebenenfalls mit gewöhnlichem alkoholhaltigem Bier verschneidet.

Die im erfindungsgemäßen Verfahren verwendete Alkoholdehydrogenase (ADH) freie Hefemutante ist vorzugsweise eine ADH negative, ADH 1-nichtrevertierbare Mutante der Hefe Saccaromyces cerevisiae.

Als "alkoholarm" wird hier in Übereinstimmung mit den einschlägigen deutschen Vorschriften ein Bier mit einem Gehalt von 0,5 bis 1,5 Gewichtsprozent Äthanol verstanden. Als "alkoholfrei" wird ein Bier mit einem Äthanolgehalt von unter 0,5, vorzugsweise unter 0,1, insbesondere unter 0,05 Gewichtsprozent (Nachweisgrenze) bezeichnet.

Beim anaeroben Stoffwechsel der erfindungsgemäß eingesetzten genetisch veränderten Hefemutanten bildet sich während der Gärung vermehrt Acetaldehyd. Acetaldehyd ist ein Zellgift und wird von der Hefe nur innerhalb gewisser Mengen toleriert. Bei Anreicherung größerer Mengen von Acetaldehyd in den Zellen oder im gärenden Substrat besteht die Gefahr einer Beeinträchtigung der Stoffwechselaktivitäten der Hefe.

Andererseits kann auch im fertigen Bier nur ein sehr geringer Gehalt an Acetaldehyd toleriert werden, wobei der Geschmacksschwellenwert bei etwa 25 T.p.M. liegt.

Der als Stoffwechselprodukt entstandene Acetaldehyd muß deshalb aus dem im Verfahren der Erfindung enthaltenen Bier entfernt werden. Dies kann beispielsweise aus dem fertigen Bier nach Beendigung der Gärung erfolgen. Im erfindungsgemäßen Verfahren ist es jedoch insbesondere wegen der schädlichen Wirkung des Acetaldehyds auf die Hefezellen bevorzugt, die Entfernung des Acetaldehyds bereits während des Gärungsprozesses durchzuführen.

Die Gärung wird deshalb im Verfahren der Erfindung vorzugsweise bei einer Temperatur oberhalb des Siedepunktes von Acetaldehyd, d.h. bei einer Temperatur über 21°C durchgeführt. Zusätzlich kann das gärende Substrat kontinuierlich oder periodisch so begast werden, daß der Acetaldehyd wirksam mit dem durchgeleiteten Gas abgeführt wird. Dabei kann bei entsprechend starker Begasung eine ausreichende Entfernung des Acetaldehyds erreicht werden, wenn einmal täglich etwa 15 bis 30 min. begast wird.

Prinzipiell können zur Begasung alle Gase verwendet werden, soweit sie den Gärprozess bzw. das fertige Produkt nicht negativ beeinflussen.

Einerseits ist es möglich, Luft zu verwenden, andererseits kann man unter streng anaeroben Bedingungen inerte Gase, insbesondere $CO_2$ verwenden. Dabei kann man einen geschlossenen Reaktor verwenden, bei dem das Gas durch geeignete, bereits bekannte Maßnahmen von den mitgeführten Stoffen befreit wird, um es erneut dem Begasungsprozeß zuzuführen (z.B. Kältetrocknung).

Im übrigen wird das Verfahren der veränderten Gärung so geführt, daß die Menge an erzeugtem Glycerin optimal für die Geschmacksharmonisierung ist. Bevorzugt ist ein Gehalt von 0,4 bis 1,5 Vol. %, insbesondere von 0,5 bis 1,2 Vol.%. Ein Zuviel an Glycerin kann durch die Herabsetzung des Endvergärungsgrades reduziert, ein Zuwenig durch Verbleiben definierter Restmengen an vergärbaren Substanzen der Bierwürze kompensiert werden.

Nachstehend wird die Herstellung von alkoholdehydrogenase-(ADH-) freien ADH1-nichtrevertierbaren Mutanten der Hefe Saccaromyces cerevisiae erläutert. (Für die Bezeichnung der ADH-Gene und ADH-Isoenzyme gilt die in (4) angegebene Nomenklatur).

1. Selektion einer ADHI-negativen Mutante (adh1) als 1mM Allylalkol-resistente Mutante auf glocosehaltigem Medium wie in Literatur (1) beschrieben. Genotyp des erhaltenen Isolats: adh1 ADH2 ADH3.

2. Ausgehend von Isolat nach 1., Selektion einer ADHII-negativen (adH2) Mutante als 1mM Allylalkohol-resistente Mutante auf glucose-freiem Medium wie in Literatur (1) angegeben.

3. Ausgehend vom Isolat nach 2., Selektion einer ADHIII-negativen (adh3) Mutante als 10 mM Allylalkohol-resistente Mutante auf glucose-freiem Medium wie in Literatur (1) angegeben. Genotyp des erhaltenen Isolats: adh1 adh2 adh3.

4. Kreuzung eines Isolats nach 3. mit einem haploiden Stamm des Genotyps ADH1 ADH2 ADH3 ura3-52 (z.B. Stamm Y27, permanent erhältlich von der Sammlung des Yeast Genetic Stock Center, Berkely/USA); Sporulation der erhaltenen diploiden Zellen; Tetradenanalyse der erhaltenen Asci; Untersuchung des ADH-Isoenzymmusters in Kulturen, die aus einzelnen Ascosporen hervorgegangen sind, wie in Literatur (1) beschrieben. Identifizierung von Isolaten des Genotyps ADH1 adh2 adh3 ura3-52, Paarungstyp MAT a oder MAT alpha, wie in (1) angegeben.

5. Konstruktion einer ADH1-Deletion (adh1-Δ) durch in vitro-Mutagenese wie in Literatur (2) angegeben.

6. Transformation eines haploiden Hefestamms ADH1 adh2 adh3 ura3-52 mit dem nach 5. erhaltenen Vektor und Identifizierung der Transformanten wie in Literatur (2) angegeben.

7. Isolierung einer ADHI-negativen Mutante ausgehend von einer Transformante nach 6. wie in Literatur (2) angegeben. Genotyp des erhaltenen Isolats:
adh1-Δ YIp5(URA ) adh1-Δ adh2 adh3 ura3-52.

8. Isolierung einer Vektor (YIp5-) freien, ADHI-negativen Mutante durch Kreuzung des nach 7. erhaltenen Isolats mit einem nach 4. erhaltenen haploiden Hefestamm des Genotyps ADH1 adh2 adh3 ura3-52; Sporulation der entstandenen diploiden Zellen; Selektion 2mM Allylalkohol-resistenter Mutanten aus der Sporulationskultur auf glucose-haltigem Medium wie unter (1) angegeben; Untersuchung des Bedürfnisses für Uracil; Isolierung Uracilbedürftiger, ADHI-negativer Segreganten; Nachweis des adh1-Δ Fragments und Nachweis des Fehlens der unter 5. verwendeten Vektorsequenz YIp5 durch die Methode von Southern (vgl. z.B. Lit.3). Genotyp der erhaltenen Isolate adh1-Δ adh2 adh3 ura3-52.

9. Um die Entstehung wachsender Mengen von Revertanten während der Fermentation zu vermeiden, sollte auch das ADH4 Gen inaktiviert werden, das in Hefen der Gattung Saccharomyces vorhanden ist und bei Inaktivierung der Gene ADH1 ADH2 und/oder ADH3 zunehmend exprimiert wird; siehe Paquin und Williamson, Mol. Cell. Biol. 6, S. 70 - 79 (1986). Ferner kann eine Mutante mit irreversibel inaktiviertem ADH3 Gen erzeugt werden, indem dieses Gen nach dem vorstehend beschriebenen Verfahren deletiert wird.

Literatur:

(1) Ciriacy, M.: Genetics of alcohol dehydrogenase in Saccharomyces cerevisiae. I. Isolation and genetic analysis of adh mutants, Mutation Research 29: 315 - 326 (1975)

(2) Williamson, V.M., Beier, D. and Young E.T., Use of transformation and meiotic gene conversion to construct a yeast strain containing a deletion in the alcohol dehydrogenase I gene, In: Genetic engineering in eukaryotes, Lurquin, P.F. and Kleinhofs, A. (ed.) Plenum Publishing Corp., N.Y., s. 21-32 (1983)

(3) Williamson, V.M., Young, E.T., Ciriacy, M., Transposable elements associated with constitutive expression of yeast alcohol dehydrogenase II,Cell 23: 605-614 (1981)

(4) Taguchi, A. K.W., Ciriacy, M. and Young, E.T., Carbon source dependence of transposable element-associated gene activiation in Saccharomyces cerevisiae, Molec. Cell. Biology 4: 61-68 (1984).

Im folgenden wird eine bevorzugte Ausführungsform des Verfahrens der Erfindung anhand eines Beispiels weiter erläutert:

Beispiel:

Eine nach dem vorstehend beschriebenen Selektionsverfahren erhaltene, ADH negative, ADH1-nicht revertierbare Mutante einer Hefe wird bei einer Temperatur von 25°C in einem Gärtank mit einem geschlossenen $CO_2$-Waschsystem mit Anstellwürze angestellt. Die Ausgangszellkonzentration liegt bei $40 \times 10^6$ Zellen/ml. Der Stammwürzegehalt der Anstellwürze beträgt etwa 11%, der wirkliche Endvergärungsgrad liegt bei etwa 67 %.

Das Bier wird praktisch endvergoren. Der Gärtank wird alle 24 Stunden zur Acetaldehydentfernung etwa 30 Min. mit $CO_2$ im Kreislaufverfahren gewaschen (etwa 30 m³/hl/h). Nach Endvergärung liegt ein Bier folgender Daten vor:
Alle Prozentangaben stellen Gewichtsprozente dar.

| | | |
|---|---|---|
| Stammwürze (%) | | 11 |
| wirklicher Extrakt (%) | | 3,7 |
| Glyceringehalt (%) | etwa | 1,5 |
| Alkohol (%) | | 0,0 |

Das Bier entspricht sensorisch nach einem normalen Reifeund Lagerprozeß einem Vollbier, das bei gleicher Ausgangswürze mit einer normalen Hefe hergestellt worden ist. Es kann zur Herstellung eines alkoholarmen Bieres in beliebiger Menge mit einem üblichen alkoholhaltigen Bier verschnitten werden.

**Ansprüche**

1. Verfahren zur Herstellung eines alkoholarmen oder alkoholfreien Bieres mit einem die Vollmundigkeit verbessernden Gehalt von 0,3 bis 2,0 Vol.% Glycerin, **dadurch gekennzeichnet,** daß man die Gärung mit einer ADH-negativen, nicht zur Alkoholbildung, dafür aber zu vermehrter Glycerinbildung befähigten Hefemutante durchführt und das erhaltene alkoholfreie Bier gegebenenfalls mit gewöhnlichem alkoholhaltigen Bier verschneidet.

2. Verfahren nach Anspruch 1, dadurch gekennzeichnet, daß man eine ADH negative, ADH1-nicht revertierbare Hefemutante einsetzt.

3. Verfahren nach Anspruch 1, dadurch gekennzeichnet, daß man eine ADH negative, ADH1-und ADH4-nicht revertierbare Hefemutante einsetzt.

4. Verfahren nach Anspruch 1, dadurch gekennzeichnet, daß man eine ADH negative, ADH1-, ADH3-und ADH4-nicht revertierbare Hefemutante einsetzt.

5. Verfahren nach Anspruch 1 bis 4, dadurch gekennzeichnet, daß man die Gärung bei einer Temperatur über 21°C durchführt.

6. Verfahren nach Anspruch 1 bis 5, dadurch gekennzeichnet, daß man die Gärung unter kontinuierlicher oder periodischer Begasung durchführt.

7. Verfahren nach Anspruch 6, dadurch gekennzeichnet, daß man die Begasung einmal täglich für 15-30 min durchführt.

8. Verfahren nach Anspruch 6 oder 7, dadurch gekennzeichnet, daß man zur Begasung Luft und/oder ein Inertgas verwendet.